# FASCICULE DE BREVET EUROPEEN

(11) **EP 0 724 600 B1**
(45) Date de publication et mention de la délivrance du brevet: **13.02.2002**
(21) Numéro de dépôt: 94931070.0
(22) Date de dépôt: 17.10.1994
(51) Int. Cl.: C07K 14/81, A61K 38/57

(54) **PROCEDE DE PREPARATION D'UN CONCENTRE D'INTER-ALPHA-TRYPSINE INHIBITEUR A USAGE THERAPEUTIQUE ET CONCENTRE OBTENU**
VERFAHREN ZUR HERSTELLUNG EINES INTER-ALPHA-TRYPSIN INHIBITOR KONZENTRATS MIT THERAPEUTISCHER ANWENDUNG UND SO ERHALTENES KONZENTRAT
PROCESS FOR PREPARING AN INTER-ALPHA-TRYPSINE INHIBITOR CONCENTRATE FOR THERAPEUTICAL USE, AND CONCENTRATE THUS OBTAINED

(30) Priorité: 18.10.1993 FR 9312346
(43) Date de publication de la demande: 07.08.1996
(73) Titulaire: ASSOCIATION POUR L'ESSOR DE LA TRANSFUSION SANGUINE DANS LA REGION DU NORD, 59000 Lille (FR)
(72) Inventeur: MICHALSKI, Catherine, F-59800 Lille (FR); MIZON, Jacques, F-59130 Lambersart (FR)
(74) Mandataire: Lepeudry-Gautherat, Thérèse
(86) Numéro de dépôt international: FR9401197
(87) Numéro de publication internationale: WO9511260

(56) Documents cités:
- EP-A- 0 237 515
- FR-A- 1 434 199
- BIOCHIMICA ET BIOPHYSICA ACTA, vol.1139, 1992 pages 122 - 132 PAOLA FINOTTI ET AL. 'PURIFICATION OF PROTEINASE-LIKE AND NA+/K+-ATPASE STIMULATING SUBSTANCE FROM PLASMA OF INSULIN-DEPENDENT DIABETICS AND ITS IDENTIFICATION AS ALPHA1-ANTITRYPSIN'

## Description

La présente invention concerne un procédé de préparation d'un concentré d'inter-alpha-trypsine inhibiteur (ITI) à partir d'une fraction de plasma humain ainsi que le concentré obtenu par ledit procédé et destiné à un usage thérapeutique.

L'ITI est un inhibiteur de sérine-protéases plasmatique. Son appellation dérive d'une part de son activité d'inhibiteur de la trypsine et, d'autre part, de sa mobilité électrophorétique située entre celle des α₁ et α₂ globulines (d'où "inter-alpha").

L'ITI appartient à la famille des "inhibiteurs de type Kunitz" (1 à 4) et comprend 3 chaînes polypeptidiques, deux lourdes H₁ et H₂ et une légère, appelée bikunine, l'ensemble étant uni par une chaîne de glycosaminoglycannes. La chaîne légère est responsable de l'activité antiprotéasique. L'ITI est une glycoprotéine présentant une masse moléculaire de 220.000 Da.

L'ITI est synthétisé par le foie et circule dans le plasma à une concentration d'environ 0,5 g/l.

L'ITI est très sensible à l'action de nombreuses protéases qui le dégradent en dérivés de plus faible poids moléculaire. Parmi ceux-ci, l'UTI (inhibiteur urinaire de la trypsine dérivé de la bikunine) se retrouve finalement dans l'urine, ce qui a permis son isolement et l'étude de ses propriétés (5, 6) ; ce fragment semble responsable de plusieurs activités biologiques de l'ITI et, en particulier, de son activité antiprotéasique.

Le rôle physiologique de l'ITI n'est pas encore totalement compris. Il présente des activités antiprotéasiques, en particulier comme inhibiteur de la trypsine et de la chymotrypsine, comme inhibiteur de l'élastase et de la cathepsine G libérées par l'activation des polynucléaires neutrophiles, comme inhibiteur de la plasmine et de la kallikréine. Il pourrait aussi avoir une activité de facteur de croissance pour les cellules endothéliales (7).

De plus, un ensemble d'études réalisées avec l'UTI indiquent que cette partie de la molécule peut jouer un rôle dans les affections à caractère inflammatoire comme la pancréatite, la polyarthrite, le choc septique et les effets secondaires associés au traitement de certains cancers. (8 à 10)

L'ITI pourrait être considéré comme un pro-inhibiteur capable de générer les fragments actifs, par exemple sous l'action des protéases libérées lors des réactions inflammatoires, lesdits fragments pouvant diffuser dans les espaces intercellulaires et participer à la protection des tissus contre les protéases.

Il pourrait donc être avantageux de disposer d'une préparation d'ITI natif , à usage thérapeutique, celui-ci pouvant présenter une demi-vie plus longue et une activité in situ plus proche de l'activité naturelle que celles de l'UTI (11).

Plusieurs publications décrivent des essais de purification de l'ITI natif à partir du plasma, soit à l'aide de sulfate d'ammonium ou de PEG puis de chromatographie d'échange d'anions; soit par chromatographie d'affinité avec un chélateur de zinc, ou sur "Blue Sepharose" (12 à 14). Les divers procédés utilisés nécessitent l'addition d'inhibiteurs de protéases, l'ITI étant très rapidement dégradé ; ces inhibiteurs sont généralement incompatibles avec une utilisation thérapeutique ; de plus, les rendements obtenus sont généralement très faibles.

La Demanderesse a donc cherché à mettre au point un nouveau procédé de purification de l'ITI, à partir du plasma humain, permettant de travailler à l'échelle industrielle et d'obtenir une molécule présentant les mêmes propriétés biologiques et structurales que la molécule native.

La Demanderesse a tiré parti d'un procédé de préparation d'un concentré de haute pureté du facteur IX de la coagulation sanguine qu'elle a mis au point (15, 16 et demande de brevet européen n° 0 317 376) et dans lequel une fraction non utilisée industriellement jusqu'à ce jour est particulièrement riche en ITI. C'est un objet de la présente invention de développer un procédé de purification de l'ITI à partir de cette fraction, sans interférer avec le rendement de purification des autres facteurs sanguins.

La Demanderesse a ainsi mis en évidence une affinité de l'ITI pour les glycosaminoglycannes sulfates (comme l'héparine) et a exploité cette propriété surprenante pour développer un procédé de séparation par chromatographie d'affinité sur colonne de glycosaminoglycanne sulfate immobilisé, par exemple sur un gel de Sepharose ® , et élution dans des conditions douces en présence de NaCl o,2 M.

La Demanderesse a montré que ce procédé était particulièrement efficace à grande échelle (c'est-à-dire à partir de volumes de plasma éventuellement supérieurs à 2.000 litres).

La Demanderesse a inclus la séparation par chromatographie d'affinité selon la présente invention dans un procédé de séparation des protéines du plasma (comme décrit plus haut). L'invention couvre également l'utilisation de la chromatographie d'affinité sur glycosaminoglycanne sulfate pour purifier l'ITI à partir de toute autre fraction résultant d'un procédé de séparation des protéines plasmatiques plus particulièrement mis au point pour optimiser la récupération d'un autre facteur.

Selon un mode de réalisation préféré, le procédé selon la présente invention comprend successivement les étapes suivantes :
- après cryoprécipitation du plasma, le surnageant est récupéré et soumis à une chromatographie d'échange d'anions sur un gel greffé de DEAE (diéthylaminoéthyle), plus particulièrement le DEAE-Sephadex A-50® , en tampon citrate de sodium, à environ 0,01 M et pH 7 ;
- après élimination des fractions contenant l'albumine, les γ-globulines, l'antithrombine III et l'α-antitrypsine, et lavage en présence de NaCl 0,23 M, l'addition de NaCl 0,5 M dans le tampon permet l'élution de la fraction PPSB (prothrombine, proconvertine, facteur de Stuart, facteur anti-hémophilique B) qui contient l'ITI ainsi que les protéines C et S;
- la fraction PPSB éluée est soumise à un traitement d'inactivation virale par solvant-détergent (TnBP-Tween),
- puis à une chromatographie d'échange d'anions sur un gel greffé de DEAE, plus particulièrement le DEAE-Sepharose ® , en tampon phosphate de sodium, à environ 6 mM et pH6, qui élimine le solvant-détergent dans le filtrat ; après lavage de la colonne en présence de NaCl 0,23 M, 2 fractions sont éluées successivement :

- la première, après addition de citrate trisodique 5mM et de NaCl 0,28 M, contient l'ITI,
- la seconde, après addition de NaCl 0,36 M, contient le F IX ;
- la fraction contenant l'ITI est soumise à une chromatographie sur héparine-Sepharose en tampon citrate de sodium, à environ 40 mM et pH 7,45, qui élimine les protéines C et S dans le filtrat et adsorbe les autres protéines ; une élution par addition de NaCl 0,2 M permet la récupération d'une fraction dont 90 % des protéines sont constitués par de l'ITI sous sa forme native ;
- après ultrafiltration et conditionnement, l'ITI est lyophilisé.

Un avantage important du procédé selon l'invention est la rapidité de son déroulement (environ 10 heures par étape) ce qui est particulièrement important pour une molécule très labile comme l'ITI.

La qualité de la préparation de concentré d'ITI obtenu par le procédé selon l'invention a été évaluée par électrophorèse et par chromatographie :
- en électrophorèse sur gel de polyacrylamide en présence ou en absence de SDS on observe une seule bande majeure de masse moléculaire environ égale à 220.000 Da. En absence de SDS on n'observe pas d'agrégats et peu de signes de dénaturation ; la bande révélée correspond à plus de 90 % des protéines ;
- par chromatographie de gel-filtration à forte pression (CLFP) on confirme la pureté supérieure à 90 % de l'ITI sous forme native.

La molécule d'ITI du concentré obtenu par le procédé selon la présente invention présente donc une structure semblable à celle de l'ITI natif c'est-à-dire qu'elle est constituée de ses 3 chaînes peptidiques, ainsi que le démontrent les électrophorèses sur gel de polyacrylamide après dissociation par la chondroïtinase.

L'activité spécifique du concentré d'ITI a été mesurée par son activité antitrypsine (dosée de manière classique sur un substrat chromogénique, le L-BAPNA) ; elle est égale à 420-500 mU/mg de protéine.

Les caractéristiques du concentré d'ITI selon l'invention en font une préparation particulièrement adaptée à un usage thérapeutique pour l'homme, notamment dans le traitement des états inflammatoires sévères.

### Références bibliographiques

- 1 - Salier JP : Inter-alpha-trypsin inhibitor: emergence of a family within the Künitz-type protease inhibitor superfamily. Trends Biochem. Sci. 1990 ; 15 : 435-439
- 2 - Balduyck M, Mizon J : l'inter-alpha-trypsine inhibiteur et ses dérivés plasmatiques et urinaires . Ann. Biol. Clin. 1991 ; 49 : 273-281
- 3 - Steinbuch M : The inter-alpha-trypsin inhibitor. Meth. Enzymol., 1976 ; 45 : 760-772
- 4 - Gebhard W, Hochstrasser K : Inter-alpha-trypsin inhibitor and its close relatives. In : Barrett, Salvesen, Proteinase inhibitors (Elsevier), 1986 ; 389-401
- 5 - Balduyck M, Hayem A, Kerckaert JP, Mizon C, Mizon J : Isolation of a human urinary trypsin inhibitor. Biochem. Biophys. Res. Comm., 1982 ; 109 : 1247-1255
- 6 - Balduyck M, Mizon C, Loutfi H, Richet C, Roussel P, Mizon J : The major urinary trypsin inhibitor is a proteoglycan. Eur. J. Biochem., 1986 ; 158 : 417-422
- 7 - McKeehan WL, Sakagani Y, Hoschi H, McKeehan KA : Two apparent human endothelial cell growth factors from human hepatoma cells are tumor-associated proteinase inhibitors. J. Biol. Chem., 1986 ; 261 : 5378-5383
- 8 - Yagi M, Tomita K, Onoda H, Konishi K, Miyazaki I : Serum granulocyte elastase and superoxyde dismutase activity after administration of protease-inhibitor to post-operative patients. J. Clin. Biochem. Nutr., 1989 ; 6 : 221-225
- 9 - Korenaga D, Orita D, Kakeji Y, Haraguchi M, Maehara Y, Sugimachi K. Prophylactic administration of urinary trypsin inhibitor prevents post-operative hyperamylasemia after R2 gastrectony in patients with gastric cancer - A prospective randomized trial. Eur. Surg. Res. 1989 ; 23 : 214-221
- 10 - Matsuo O, Tanaka S, Kikuchi H. Effect of urinary trypsin inhibitor on osteoarthritis. Thromb. Res. 1988 ; 52 : 237-245
- 11 - Jönsson-Berling BM, Ohlsson K. Distribution and elimination of intravenouly injected urinary trypsin inhibitor. Scand. J. Clin. Lab. Invest. 1991 ; 51 : 549-557
- 12 - Salier JP, Martin JP, Lambin P, McPhee H., Hochstrasser K. Purification of the human sérum inter-alpha-trypsin inhibitor by zinc-chelate and hydrophobic interaction chromatographies. Anal. Biochem. 1980 ; 109 : 273-283
- 13 - Lambin P, Fine JM, Steinbuch M. Nouvelles données sur une antiprotéase du plasma humain : l'inter-alpha-trypsine inhibiteur. Rev. Fr. Transf. Imnunohematol. 1975 ; XVIII : 385-400
- 14 - Enghild JJ, Thogersen IB, Pizzo SV, Salvesen G. Analysis of ITI and a novel trypsin inhibitor, pre-alpha-trypsin inhibitor from human plasma. J. Biol. Chem. 1989 ; 264 : 15975-15981
- 15 - Michalski C, Bal F, Burnouf T, Goudemand M. Large scale production and properties of a solvent-detergent treated factor IX concentrate from human plasma. Vox Sang. 1988 ; 55 : 202-210
- 16 - Burnouf T, Michalski C, Goudemand M, Huart JJ. Properties of a highly purified human plasma factor IX:c therapeutic concentrate prepared by conventional chromatography. Vox Sang 1989 ; 57 : 225-232

Les exemples suivants illustrent un mode de réalisation et de caractérisation de l'invention, sans toutefois en limiter la portée.

### Exemple I

Un lot de plasma, destiné à la préparation de concentré de Facteur IX de haute pureté est traité de manière habituelle jusqu'à l'obtention du PPSB (Prothrombine, Proconvertine, facteur de Stuart et facteur IX ou antihémophilique B).

Pour mémoire, le plasma frais ou congelé-décongelé est soumis à une cryoprécipitation, qui élimine le F VIII, le facteur von Willebrand, le fibrinogène et la fibronectine. Le surnageant (de 1000 à 1.500 litres) est récupéré et soumis à une chromatographie sur gel de DEAE-Sephadex ® (1,5 g par litre de surnageant) en tampon citrate de sodium 0,01 M à pH 7,0. L'albumine, les γ-globulines, l'AT III et l'α₁-antitrypsine se retrouvent dans le filtrat. La colonne est lavée avec le même tampon additionné de NaCl 0,23 M puis la fraction PPSB est éluée par addition de NaCl 0,5 M dans le tampon. Cette fraction contient jusqu'à 20 à 40 % en poids des protéines d'ITI (qui était jusqu'à présent considéré comme un contaminant majeur). Cette fraction est ajustée à 40 g/l de protéines et à une osmolarité de 290 mosm/l à pH7 et additionnée de lysine 2,5 g/l.

Cette fraction est soumise à un traitement d'inactivation virale par contact avec du TnBP/Tween (0,3 % tri(n-butyl)phosphate - 1 % Tween 80, en concentration finale) à 24°C pendant 6 heures.

Cette fraction est ensuite soumise à une chromatographie sur gel de DEAE- Sepharose ® FF en tampon phosphate de sodium 6mM à pH 6,0. On injecte 10 à 12 litres du PPSB sur une colonne contenant environ 18 litres de DEAE-Sepharose. Le mélange TnBP/Tween est éliminé dans le filtrat. La colonne est lavée deux fois en tampon phosphate de sodium 5 mM - citrate trisodique 5 mM, à pH 6,0, additionné de NaCl 0,16 M puis de NaCl 0,23 M. Puis la fraction contenant l'ITI est éluée par augmentation de la concentration en NaCl à 0,28 M, dans le même tampon. Enfin, l'augmentation de la concentration en NaCl à 0,36 M permet l'élution de la fraction enrichie en FIX qui sera encore purifiée par une chromatographie supplémentaire.

La fraction enrichie en ITI contient également des protéines C et S, du Facteur X, du C4 et du kininogène de haut poids moléculaire.

Dans les autres fractions on trouve un peu d'ITI dégradé, de MM 120.000 ou de formes agrégées, qui sont éliminées.

Cette fraction enrichie en ITI est ajustée à 40 g/l de protéines à une osmolarité de 100 mosm/l et à un pH de 7,45. Elle est alors soumise à une chromatographie sur héparine-Sepharose® en tampon citrate trisodique 40 mM à pH 7,45. On utilise environ 2 litres de la fraction concentrée pour une colonne de 18 litres de gel. Les protéines C et S sont éliminées dans le filtrat ; les autres composants de cette fraction sont adsorbés sur la colonne. La colonne est lavée avec le même tampon additionné de NaCl 0,05 M, ce qui élimine le Facteur X et les formes dégradées de l'ITI. Ensuite l'ITI est élué par addition de NaCl 0,2 M dans le tampon. La fraction éluée contient plus de 90 % (en poids de protéines) d'ITI.

La concentration de NaCl ne doit pas dépasser 0,2 M pour éviter la formation d'agrégats.

Après concentration à environ 8 g/l, l'ITI est ultrafiltré dans du tampon citrate 1 g/l - NaCl 9 g/l éventuellement additionné d'arginine 4,5 g/l et de lysine 4,5 g/l, à pH 7. Le concentré est ajusté à une osmolarité d'environ 300 mosm/l à pH 7, filtré stérilement, réparti en flacons et lyophilisé.

### Exemple II Caractéristiques biochimiques du concentré d'ITI obtenu.

1) L'activité anti-trypsine est dosée de manière classique sur le substrat chromogénique L-BAPNA (α-N-benzoyl-L-arginine-p-nitroanilide) :
   l'activité du concentré est de 3.500-4.500 mU/ml
   l'activité spécifique est de 420-500 mU/mg protéine.

   Les taux résiduels de protéine C et S et de facteur X sont inférieurs à 0,5 UI/ml. Des traces de C₄ sont décelées par la technique d'Ouchterlony, et représentent moins de 0,1 mg/ml (par dosage immunonéphélémétrique).
2) L'électrophorèse sur acétate de cellulose fait apparaître une seule bande, de mobilité électrophorétique située entre celle des α₁ et α₂ globulines.
3) L'électrophorèse sur gel de polyacrylamide, colorée au bleu de Coomassie, fait apparaître une seule bande majeure de masse moléculaire de 220.000 daltons, représentant au moins 95 % des protéines soumises à l'analyse (les standards utilisés comme références de masse moléculaire étaient la thyroglobuline, 660.000, la ferritine, 440.000, la catalase, 232.000, l'aldolase, 150.000 et la serumalbunine, 67.000).
4) Après digestion de l'ITI par la chondroïtinase, l'analyse des électrophorèses sur gel de polyacrylamide en présence de SDS montre que la bande majeure à 220.000 a complètement disparu et qu'on visualise les 2 chaînes lourdes, H₁ à 96.000 et H₂ à 86.000, et la bikunine (sans GAG) à 20.000. (Les standards utilisés comme références de masse moléculaire étaient la myosine, 200.000 ; la β-glactosidase, 116.000 ; la phosphorylase B, 97.000 ; la serumalbumine bovine, 67.000 et l'ovalbumine, 45.000.
5) L'homogénéité de la préparation a été vérifiée par chromatographie de tamisage moléculaire.

### Exemple III - Innocuité du concentré d'ITI obtenu, dans les modèles animaux.

Le concentré lyophilisé et reconstitué (à un contenu en protéines totales de 8 g/l) a été contrôlé dans divers modèles animaux.
1) La toxicité éventuelle a été étudiée sur 10 souris, par injection intraveineuse de 0,5 ml par 20 g de poids corporel. Après 7 jours aucune mortalité ni aucun effet létal n'est observé.
2) La thrombogénicité potentielle a été évaluée dans le modèle de Wessler de stase sur lapins, par injection intraveineuse de 10 ml/kg. On n'a observé aucune formation de caillot sanguin, ce qui montre l'absence de composants thrombogènes de la cascade de la coagulation.
3) L'absence de contaminants tels que des substances vasoactives (comme des agrégats protéiques ou des peptides libérés par le kininogène-HMW) a été contrôlée sur des rats, par la mesure du rythme cardiaque et de la pression sanguine, après injection intraveineuse de 4 ml/kg à 0,1 ml/sec. Ces 2 paramètres restent inchangés (variation de ±2 %) après l'injection.
4) L'absence de pyrogénicité a été contrôlée sur lapins comme prescrit dans la Pharmacopée européenne, à une dose de 2 ml/kg.

## Revendications

1. Procédé de préparation d'un concentré d'inter-alpha-trypsine inhibiteur (ITI) à usage thérapeutique, à partir d'une fraction de plasma humain enrichie en ITI, **caractérisé en ce qu'**il comprend une étape de chromatographie d'affinité sur un glycosaminoglycanne sulfate, tel que l'héparine, immobilisé sur un gel.

2. Procédé selon la revendication 1, **caractérisé en ce que** la fraction de plasma enrichie en ITI est obtenue par deux étapes successives de séparation par chromatographie sur gels échangeurs d'anions.

3. Procédé selon la revendication 1 ou 2, **caractérisé en ce qu'**il comprend un traitement d'inactivation virale par solvant-détergent avant la deuxième étape de chromatographie.

4. Procédé selon l'une quelconque des revendications 1 à 3, **caractérisé en ce qu'**il comprend successivement les étapes suivantes :
a) le surnageant du plasma cryoprécipité est soumis à une chromatographie d'échange d'anions sur gel greffé de DEAE, en tampon citrate de sodium, à environ 0,01 mM et pH 7 ;
b) la fraction contenant le PPSB et l'ITI est éluée de la colonne de chromatographie par l'addition de NaCl 0,5 M dans le tampon.
c) la fraction éluée en b) est soumise à un traitement d'inactivation virale par solvant-détergent puis à une chromatographie d'échange d'anions sur gel greffé de DEAE, en tampon phosphate de sodium, à environ 6 mM et pH 6 ;
d) la fraction contenant l'ITI est éluée de la colonne de chromatographie par l'addition de citrate trisodique 5 mM et de NaCl 0,28 M dans le tampon ;
e) la fraction éluée en d) est soumise à une chromatographie sur héparine immobilisée, plus particulièrement sur héparine-Sepharose, en tampon citrate de sodium, à environ 40 mM et pH 7,45 ;
f) la fraction contenant l'ITI et dans laquelle celui-ci représente 90 % des protéines est éluée de la colonne de chromatographie par l'addition de NaCl 0,2 M dans le tampon ;
g) la fraction éluée en f) est concentrée par ultrafiltration, conditionnée et lyophilisée.

5. Concentré d'ITI humain à usage thérapeutique obtenu par le procédé selon l'une quelconque des revendications 1 à 4, **caractérisé en ce que** l'ITI présente une structure semblable à celle de l'ITI plasmatique natif, c'est-à-dire qu'il est constitué de 3 chaînes peptidiques et qu'il a une masse moléculaire d'environ 220 KDa.

6. Concentré d'ITI humain selon la revendication 5 **caractérisé en ce qu'**il présente une activité spécifique égale à 420-500 mU/mg de protéine.

## Claims

1. Method for preparing an inter-alpha-trypsine inhibitor concentrate (ITI) for therapeutic use, from a human plasma fraction enriched in ITI, **characterized in that** it comprises one step of affinity chromatography on a glycosaminoglycanne sulfate, such as heparin, immobilised on a gel.

2. Method according to claim 1, **characterized in that** the plasma fraction enriched in ITI is obtained by two successive separation steps by anion exchange chromatography.

3. Method according to claim 1, **characterized in that** it comprises a viral inactivation treatment by solvent-detergent prior to the second chromatography step.

4. Method according to any one of claims 1 to 3, **characterized in that** it successively comprises the following steps :
a) the supernatant of the cryoprecipitated plasma is subjected to an anion exchange chromatography on a gel grafted with DEAE, in sodium citrate buffer, at about 0,01 mM and pH 7;
b) the PPSB and ITI containing fraction is eluted from the chromatography column by adding 0.5 M NaCl into the buffer;
c) the fraction eluted in b) is subjected to a viral inactivation treatment by solvent-detergent then to an anion exchange chromatography on a gel grafted with DEAE, in sodium phosphate buffer, at about 6 mM and pH 6;
d) the ITI containing fraction is eluted from the chromatography column by adding 5 mM trisodic citrate and 0.28 M NaCl into the buffer;
e) the fraction eluted in d) is subjected to a chromatography on immobilised heparin, more particularly heparin-Sepharose, in sodium citrate buffer, at about 40 mM and pH 7.45;
f) the ITI containing fraction wherein said ITI represents 90 % of the proteins is eluted from the chromatography column by adding 0.2 M NaCl into the buffer;
g) the fraction eluted in f) is concentrated by ultrafiltration, dispensed and freeze-dried.

5. Human ITI concentrate for therapeutic use obtained by the method according to any one of claims 1 to 4, **characterized in that** ITI has a structure similar to that of the native plasmatic ITI, that is to say it is constituted of three peptidic chains and it has a molecular mass of about 220 Kda.

6. Human ITI concentrate according to claim 5 **characterized in that** it has a specific activity of 420-500 mU/mg of protein.

## Patentansprüche

1. Verfahren zur Herstellung eines Konzentrats von Inter-Alpha-Trypsin-Inhibitor (ITI) zum therapeutischen Gebrauch aus einer an ITI angereicherten menschlichen Plasmafraktion, **dadurch gekennzeichnet, dass** es einen Schritt der Affinitätschromatografie auf einem Glykosaminoglykansulfat, etwa Heparin, umfasst, das auf einem Gel immobilisiert ist.

2. Verfahren nach Anspruch 1, **dadurch gekennzeichnet, dass** die an ITI angereicherte Plasmafraktion erhalten wird durch zwei aufeinander folgende Trennungsschritte durch Chromatografie auf Anionenaustauschergelen.

3. Verfahren nach Anspruch 1 oder 2, **dadurch gekennzeichnet, dass** es eine Behandlung zur viralen Inaktivierung durch Solvens-Detergens vor dem zweiten Schritt der Chromatografie umfasst.

4. Verfahren nach einem der Ansprüche 1 bis 3, **dadurch gekennzeichnet, dass** es nacheinander die folgenden Schritte umfasst:
a) der Überstand des bei tiefer Temperatur gefällten Plasmas wird im Natriumcitratpuffer mit ungefähr 0,01 mmol/l und pH 7 einer Anionenaustauschchromatografie auf mit DEAE gepfropftem Gel unterzogen;
b) die das PPSB und das ITI enthaltende Fraktion wird durch die Zugabe von 0,5 mol/l NaCl in den Puffer von der chromatografischen Kolonne eluiert;
c) Die in b) eluierte Fraktion wird einer Behandlung zur viralen Inaktivierung durch Solvens-Detergens, dann im Natriumphosphatpuffer bei ungefähr 6 mmol/l und pH 6 einer Anionenaustauschchromatografie auf mit DEAE gepfropftem Gel unterzogen;
d) die das ITI enthaltende Fraktion wird durch die Zugabe von 5 mmol/l Trinatriumcitrat und von 0,28 mol/l NaCl in den Puffer von der chromatografischen Kolonne eluiert;
e) die in d) eluierte Fraktion wird in einem Natriumcitratpuffer mit ungefähr 40 mmol/l und pH 7,45 einer Chromatografie auf immobilisiertem Heparin, insbesondere auf Heparin-Sepharose, unterzogen;
f) die das ITI enthaltende Fraktion, in welcher dieses 90% der Proteine darstellt, wird durch die Zugabe von 0,2 mol/l NaCl in den Puffer von der chromatografischen Kolonne eluiert;
g) die in f) eluierte Fraktion wird konzentriert durch Ultrafiltration, konditioniert und gefriergetrocknet.

5. Konzentrat von menschlichem ITI zum therapeutischen Gebrauch, erhalten durch das Verfahren nach einem der Ansprüche 1 bis 4, **dadurch gekennzeichnet, dass** das ITI eine Struktur aufweist, die zu der des nativen plasmatischen ITI gleichartig ist, d.h. es besteht aus 3 Peptidketten und hat eine molekulare Masse von ungefähr 220 KDa.

6. Konzentrat von menschlichem ITI nach Anspruch 5, **dadurch gekennzeichnet, dass** es eine spezifische Aktivität besitzt, die gleich 420 - 500 mE pro mg Protein ist.
